# FASCICULE DE BREVET EUROPEEN

(11) **EP 0 872 259 B1**
(45) Date de publication et mention de la délivrance du brevet: **11.09.2002**
(21) Numéro de dépôt: 98400704.7
(22) Date de dépôt: 26.03.1998
(51) Int. Cl.: A61M 27/00, A61M 25/00

(54) **Drain chirurgical souple pourvu d'une pluralité de conduits individuels**
Flexibles chirurgisches Drainagerohr mit mehreren individuellen Leitungen
Flexible chirurgical drain with a plurality of individual ducts

(30) Priorité: 14.04.1997 FR 9704532
(43) Date de publication de la demande: 21.10.1998
(73) Titulaire: PORGES, 92350 Le Plessis-Robinson (FR)
(72) Inventeur: Ismael, Bernard, 75019 Paris (FR)
(74) Mandataire: Bonnetat, Christian

(56) Documents cités:
- EP-A- 0 332 366
- EP-A- 0 385 168
- EP-A- 0 386 408
- DE-A- 2 820 239
- DE-U- 8 815 869
- US-A- 4 406 656

## Description

La présente invention concerne les drains chirurgicaux souples à plusieurs conduits individuels, destinés au drainage de cavités biologiques, telles que plaies, abcès, etc...

On connaît déjà des drains chirurgicaux de ce type (par example de EP-A-0 332 366), dans lesquels une pluralité de conduits individuels parallèles sont deux à deux accolés et solidaires, de façon à former une bande plate, à une seule couche de conduits, dont l'épaisseur est déterminée uniquement par le diamètre desdits conduits et dont la largeur est déterminée par le nombre et par le diamètre desdits conduits. Ainsi, si le nombre des conduits individuels est élevé (par exemple de l'ordre de 5 à 10), la largeur d'un tel drain est grande.

Un tel drain à plusieurs conduits en forme de bande plate peut être mis en place dans la cavité à drainer en introduisant son extrémité distale dans celle-ci. On remarquera qu'à cause de la largeur importante dudit drain, il est indispensable que le chirurgien pratique une large incision pour donner accès ou élargir l'accès à ladite cavité. Cependant, le plus souvent, les conduits individuels sont séparés les uns des autres à l'extrémité distale dudit drain pour pouvoir être largement répartis dans ladite cavité et, donc améliorer le drainage. Dans ce cas, le mode de mise en place simple, mentionné ci-dessus, ne peut être utilisé. On est donc alors obligé d'introduire le drain dans ladite cavité par son extrémité proximale, après réalisation de l'incision mentionnée ci-dessus, puis de ressortir à l'extérieur du patient ladite extrémité proximale à travers une autre incision, et enfin de tirer cette extrémité proximale jusqu'à ce que les conduits séparés de l'extrémité distale du drain se trouvent dans ladite cavité. Le chirurgien pratique donc alors deux larges incisions.

Ainsi, ces drains connus à plusieurs conduits en forme de bande plate présentent l'inconvénient de nécessiter la réalisation d'une ou de deux larges incisions. De plus, étant donné leur largeur, il en résulte d'une part, que leur retrait après drainage de la cavité est souvent traumatisant pour le patient et, d'autre part, qu'ils ne peuvent être utilisés en coeliochirurgie. Enfin, il est difficile, sinon impossible, de connecter leur extrémité proximale à un dispositif extérieur, par exemple pour injecter un liquide de lavage dans la cavité et/ou pour aspirer des liquides hors de la cavité, à travers un conduit individuel.

Pour tenter de remédier à ces inconvénients, certains chirurgiens ont pensé à rouler le drain sur lui-même autour de sa direction longitudinale pour en faire un rouleau à section spiralée, qu'ils peuvent introduire et/ou sortir par une plus petite incision ou, en coelioscopie, par un trajet laissé par un trocart. Cependant, si un tel mode opératoire permet d'éviter certains des inconvénients précités, il ne les élimine pas tous : par exemple, la connexion à un dispositif de lavage ou d'aspiration reste impossible. De plus, il comporte ses propres inconvénients, tels que la stagnation de liquides biologiques entre les spires du rouleau ou la tendance naturelle au déroulement et à l'expansion radiale, ce qui rend difficile l'extraction dudit drain.

La présente invention a pour objet de remédier à tous les inconvénients des drains plats connus, rappelés ci-dessus.

A cette fin, selon l'invention, le drain chirurgical, comportant une pluralité de conduits individuels deux à deux accolés et solidaires, est remarquable en ce qu'il présente la forme d'un tube dont la paroi est constituée par lesdits conduits individuels accolés. De préférence, lesdits conduits individuels accolés délimitent entre eux une lumière longitudinale centrale. Une telle lumière longitudinale centrale peut présenter une section de toute forme désirée, par exemple circulaire ou hexagonale telle que mentionnée ci-dessous. Elle peut bien entendu être utilisée comme chacun des conduits individuels.

Ainsi, comme pour le drain plat roulé longitudinalement sur lui-même, on évite les larges incisions. Mais, de plus, dans le drain conforme à la présente invention :
. le diamètre extérieur du drain est constant, ce qui facilite le passage à l'intérieur d'un trocart ;
. la lumière longitudinale centrale présente une section constante et aucune rétention de liquides biologiques n'est possible ;
. la forme tubulaire permet la réalisation facile des raccords de connexion de l'extrémité proximale à un dispositif extérieur, par exemple pour l'obtention d'un drainage aspiré, avec ou sans lavage.

Bien entendu, de par sa forme tubulaire, le drain conforme à l'invention peut être utilisé, aussi bien en chirurgie classique qu'en coeliochirurgie.

On notera que lesdits conduits individuels accolés pourraient présenter une forme hélicoïdale autour de l'axe du drain. Cependant, dans un mode de réalisation préféré, lesdits conduits individuels accolés sont rectilignes, et donc parallèles entre eux et à l'axe longitudinal dudit drain.

De même, lesdits conduits individuels accolés pourraient être différents les uns des autres, par exemple en ce qui concerne leur diamètre. Il est toutefois avantageux qu'ils soient tous identiques.

Afin de pouvoir séparer lesdits conduits individuels accolés les uns aux autres à l'extrémité distale dudit drain, on prévoit entre eux des lignes longitudinalement déchirables.

Dans un mode de réalisation préféré, le drain de l'invention comporte six conduits individuels accolés identiques délimitant entre eux une lumière longitudinale centrale dont la section est un hexagone régulier à côtés curvilignes concaves.

Les figures du dessin annexé feront bien comprendre comment l'invention peut être réalisée. Sur ces figures, des références identiques désignent des éléments semblables.

La figure 1 est une vue en perspective du mode de réalisation préféré du drain tubulaire conforme à la présente invention.

La figure 2 montre la section du drain tubulaire de la figure 1.

La figure 3 illustre schématiquement le drain tubulaire des figures 1 à 2 en position de drainage d'une cavité biologique.

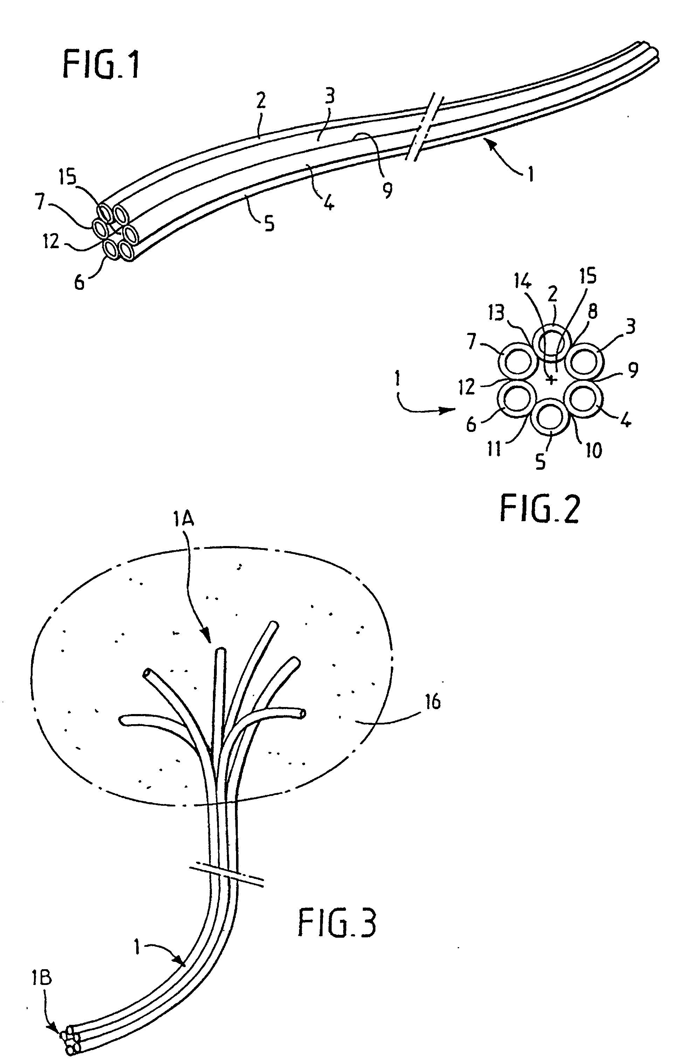

Le drain 1, conforme à la présente invention et montré sur les figures 1 et 2, est constitué par une pluralité de conduits individuels rectilignes identiques 2 à 7, deux à deux accolés le long de lignes longitudinales rectilignes 8 à 13. Dans chaque section dudit drain 1, les conduits individuels 2 à 7 sont régulièrement répartis autour du centre 14 de ladite section, de sorte que ledit drain 1 présente la forme d'un tube dont la paroi est constituée par lesdits conduits individuels accolés qui délimitent entre eux, sur toute la longueur dudit drain 1, une lumière longitudinale intérieure 15 à section constante en forme d'hexagone régulier à côtés curvilignes concaves.

Le drain 1 conforme à la présente invention est avantageusement obtenu par extrusion d'une matière synthétique souple biocompatible, telle que par exemple un silicone.

Avantageusement, les lignes de liaison longitudinales 8 à 13 entre les conduits individuels 2 à 7 sont déchirables, de sorte que, pour drainer plus efficacement une cavité biologique 16 (voir la figure 3), lesdits conduits individuels 2 à 7 peuvent être séparés les uns des autres à l'extrémité distale lA dudit drain 1 disposée dans ladite cavité biologique 16 (plaie, abcès, etc ...). Ainsi, lesdits conduits individuels 2 à 7 peuvent se répartir à l'intérieur de ladite cavité 16 et drainer en parallèle des zones différentes de celle-ci.

Bien que sur les figures 1 à 3 on ait représenté un mode de réalisation préféré à six conduits individuels identiques et rectilignes 2 à 7, il va de soi que le drain conforme à la présente invention pourrait comporter un nombre différent de conduits individuels, semblables ou non.

On remarquera que, du fait de la forme tubulaire du drain 1, il est commode de réaliser un embout (non représenté) susceptible de coopérer avec l'extrémité proximale 1B dudit drain, pour connecter les conduits individuels à des dispositifs d'injection de liquide de lavage ou d'aspiration, ou même à une poche de colostomie.

## Revendications

1. Drain chirurgical (1) comportant une pluralité de conduits individuels (2 à 7), **caractérisé en ce que** ladite pluralité de conduits présente la forme d'un tube dont la paroi est constituée par lesdits conduits individuels (2 à 7), qui sont accolés et solidaires les uns des autres le long de lignes longitudinales (8 à 13) déchirables.

2. Drain selon la revendication 1,
**caractérisé en ce que** lesdits conduits individuels accolés (2 à 7) délimitent entre eux une lumière longitudinale centrale (15).

3. Drain selon la revendication 2,
**caractérisé en ce que** ladite lumière longitudinale centrale (15) présente une section circulaire.

4. Drain selon l'une des revendications 1 à 3,
**caractérisé en ce que** lesdits conduits individuels accolés (2 à 7) sont hélicoïdaux.

5. Drain selon l'une des revendications 1 à 3,
**caractérisé en ce que** lesdits conduits individuels accolés (2 à 7) sont rectilignes.

6. Drain selon l'une des revendications 1 à 5,
**caractérisé en ce que** lesdits conduits individuels accolés (2 à 7) sont identiques.

7. Drain selon l'une des revendications 1 à 6,
**caractérisé en ce que** qu'il comporte six conduits individuels (2 à 7) et **en ce que** la section de la lumière longitudinale centrale (15) est un hexagone régulier à côtés curvilignes concaves.

## Patentansprüche

1. Chirurgischer Drän (1), welcher eine Anzahl von Einzelleitungen (2 bis 7) aufweist, **dadurch gekennzeichnet, dass** die besagte Anzahl von Einzelleitungen die Form einer Röhre aufweist, deren Wandung aus den besagten Einzelleitungen (1 bis 7) besteht, welche längs von sich in Längsrichtung erstreckenden auftrennbaren Linien (8 bis 13) aneinander befestigt und untereinander fest verbunden sind.

2. Drän nach Anspruch 1, **dadurch gekennzeichnet, dass** die besagten, aneinander befestigten Einzelleitungen (2 bis 7) zwischen sich einen zentralen Längskanal (15) begrenzen.

3. Drän nach Anspruch 2, **dadurch gekennzeichnet, dass** der besagte zentrale Längskanal (15) einen kreisförmigen Querschnitt aufweist.

4. Drän nach einem der Ansprüche 1 bis 3, **dadurch gekennzeichnet, dass** die besagten, aneinander befestigten Einzelleitungen (2 bis 7) schraubenförmig sind.

5. Drän nach einem der Ansprüche 1 bis 3, **dadurch gekennzeichnet, dass** die besagten, aneinander befestigten Einzelleitungen (2 bis 7) geradlinig sind.

6. Drän nach einem der Ansprüche 1 bis 5, **dadurch gekennzeichnet, dass** die besagten, aneinander befestigten Einzelleitungen (2 bis 7) identisch sind.

7. Drän nach einem der Ansprüche 1 bis 6, **dadurch gekennzeichnet, dass** er sechs Einzelleitungen (2 bis 7) aufweist, und dadurch, dass der Querschnitt des zentralen Längskanals (15) ein regelmäßiges Sechseck mit konkav gekrümmten Seiten ist.

## Claims

1. Surgical drain (1) comprising a plurality of individual ducts (2 to 7), **characterized in that** the said plurality of ducts is in the form of a tube whose wall is composed of the said individual ducts (2 to 7), which are juxtaposed and joined to each other side by side along tearable longitudinal lines (8 to 13).

2. Drain according to Claim 1, **characterized in that** the said individual juxtaposed ducts (2 to 7) define a central longitudinal passage (15) between themselves.

3. Drain according to Claim 2, **characterized in that** the said central longitudinal passage (15) is of circular section.

4. Drain according to one of Claims 1 to 3, **characterized in that** the said individual juxtaposed ducts (2 to 7) are helical.

5. Drain according to one of Claims 1 to 3, **characterized in that** the said individual juxtaposed ducts (2 to 7) are straight.

6. Drain according to one of Claims 1 to 5, **characterized in that** the said individual juxtaposed ducts (2 to 7) are identical.

7. Drain according to one of Claims 1 to 6, **characterized in that** it comprises six individual ducts (2 to 7) and **in that** the cross section of the central longitudinal passage (15) is a regular hexagon with concave curved sides.
